# EUROPEAN PATENT SPECIFICATION

(11) **EP 4 045 134 B1**
(45) Date of publication and mention of the grant of the patent: **09.12.2026**
(21) Application number: 20728306.0
(22) Date of filing: 04.05.2020
(51) Int. Cl.: A61N 1/05, A61N 1/372, A61N 1/36, A61N 1/375, A61N 1/378

(54) **BIASED NEUROMODULATION LEAD**
VORGESPANNTE NEUROMODULATIONSLEITUNG
SONDE DE NEUROMODULATION PRÉCONTRAINTE

(30) Priority: 15.10.2019 US 201962915194 P
(43) Date of publication of application: 24.08.2022
(73) Proprietor: XII Medical, Inc., Union City, CA 94587 (US)
(72) Inventor: CAPARSO, Anthony V., Ridgeville, Ohio 44039 (US); NICKOLS, Josh, Louisville, Kentucky 40205 (US); SOOSE, Ryan, Sewickley, PA (US)
(74) Representative: Forresters IP LLP
(86) International application number: PCT/US2020/031279
(87) International publication number: WO 2021/076188

(56) References cited:
- WO-A1-2017/173433
- JP-A- 2013 208 182
- US-A- 5 609 621
- US-A1- 2009 270 960
- US-A1- 2010 179 562
- US-A1- 2010 292 769
- US-A1- 2011 071 606
- US-A1- 2014 379 049
- US-A1- 2017 087 360

## Description

### TECHNICAL FIELD

The present disclosure relates to a neuromodulation lead.

### BACKGROUND

Upper airway sleep disorders (UASDs) or sleep disordered breathing (SDB) are conditions that occur in the upper airway that diminish sleep time and sleep quality, resulting in patients exhibiting symptoms that include daytime sleepiness, tiredness and lack of concentration. Obstructive sleep apnea (OSA), the most common type of UASD, is characterized by cessation of airflow because of upper airway obstruction despite simultaneous respiratory effort. The respiratory effort continues despite obstruction until the individual is aroused from sleep. During sleeping the genioglossus muscle and other muscles that hold the airway patent relax, causing the mandible and the tongue to move posteriorly, which decreases upper airway volume. The obstruction causes a decrease in oxygen blood level, leading to increased respiratory drive and this cycle continues until the patient is aroused.

OSA is highly prevalent, affecting one in five adults in the United States. One in fifteen adults has moderate to severe OSA requiring treatment. OSA is the most common type of sleep apnea. Untreated OSA results in reduced quality of life measures and increased risk of disease including hypertension, stroke, heart disease, etc. Continuous positive airway pressure (CPAP) is a standard treatment for OSA. While CPAP is non-invasive and highly effective, it is not well tolerated by all patients and has several side effects. Patient compliance and/or tolerance for CPAP is often reported to be between 40% and 60%. Surgical treatment options for OSA, such as anterior tongue muscle repositioning, orthognathic bimaxillary advancement, uvula-palatal-pharyngoplasty, and tracheostomy are available too. However, they tend to be highly invasive (result in structural changes), irreversible, and have poor and/or inconsistent efficacy. Even the more effective surgical procedures are undesirable because they usually require multiple invasive and irreversible operations, they may alter a patient's appearance (e.g., maxillo-mandibular advancement), and/or they may be socially stigmatic (e.g., tracheostomy) and have extensive morbidity.

US 2009/270960 discloses systems, devices and methods for delivering electrodes for spinal cord stimulation.

US 2014/379049 discloses a neuromodulation device including a carrier configured for implantation in a subject, the carrier including a first elongated arm and a second elongated arm, each extending from a central portion of the carrier. The device further includes a first pair of modulation electrodes arranged on the first elongated arm, a second pair of modulation electrodes arranged on the second elongated arm. The carrier is sized and shaped for implantation in a vicinity of a hypoglossal nerve to be modulated such that the first pair of modulation electrodes is located to modulate a first hypoglossal nerve on a first side of the subject and the second pair of modulation electrodes is located to modulate a second hypoglossal nerve on a second side of the subject.

US 2017/087360 discloses devices and methods for treating sleep apnea, in particular a neural stimulation device comprising a single lead having a first and a second end that lacks a mechanical connection to a mounting retainer. The lead includes a first and a second electrode positioned along the single lead and is placed in the base of the tongue near a first and/or second hypoglossal nerve. The lead is disposed perpendicular to a longitudinal axis of a patient's tongue such that the first electrode is near a first hypoglossal nerve and the second electrode is near a second hypoglossal nerve.

### SUMMARY

The invention is defined in the claims. Methods described hereinafter are only of exemplary nature and presented for better understanding the present invention which is defined by the appended claims. Provided herein are neuromodulation leads. A neuromodulation lead can be used to stimulate the distal hypoglossal nerve trunk as well as distal branches of the hypoglossal nerve trunk that innervate the horizontal fibers and/or the oblique fibers within the genioglossus muscle as well as other muscles of the anterior lingual musculature. The application of stimulation to the hypoglossal nerve trunk and hypoglossal nerve distal fibers can enable airway maintenance during sleep for the treatment of SDB, such as obstructive sleep apnea (OSA). The neuromodulation lead comprises a plurality of electrodes and is be configured or biased such that when positioned at the target site, i.e., between the geniohyoid and genioglossus muscles, the lead and the electrodes mirror the anatomy of the hypoglossal nerve bilaterally as the nerve approaches and starts to branch into the genioglossus muscle. In addition, or alternatively, the lead can be configured or biased to exert slight pressure on the genioglossus muscle and/or the hypoglossal nerve in an implanted configuration of the lead.

The invention provides a neuromodulation lead configured to be placed between a geniohyoid muscle and a genioglossus muscle comprising a lead body having a left portion, a right portion, and an intermediate portion therebetween; a left set of electrodes disposed on the left portion of the lead body; and a right set of electrodes disposed on the right portion of the lead body, wherein the left portion, the right portion, the intermediate portion and the locations of the left and right sets of electrodes respectively on the left and right portions of the lead body are configured to align the left set of electrodes and the right set of electrodes with the left nerve branches and the right nerve branches of a hypoglossal nerve, respectively, when the neuromodulation lead is fully deployed, and wherein the intermediate portion of the lead body is biased towards an inferior position relative to the left and right sets of electrodes when the neuromodulation lead is fully deployed, such that the intermediate portion is configured to push the left and the right sets of electrodes further cranially into the genioglossus muscle.

The lead body can be biased towards a substantially omega shape when fully deployed.

The location of the left and right set of electrodes on the respective left and right portion of the lead body can be based on the location of an electrical stimulation target site comprising the hypoglossal nerve trunk, distal branches of the hypoglossal nerve, nerve branches that innervate horizontal fibers within the genioglossus muscle, nerve branches that innervate oblique fibers within the genioglossus muscle, or suitable combinations thereof when the neuromodulation lead is fully deployed.

### BRIEF DESCRIPTION OF DRAWINGS

FIG. 1 is a cut-away lateral view of the anterior lingual musculature, the hypoglossal nerve and exemplary terminal branches thereof, and surrounding bony structure, including the mandible, of a human.
FIG. 2 is a schematic transverse view of the geniohyoid muscle, the genioglossus muscle and the mylohyoid muscle of a human with the mylohyoid muscle oriented inferiorly and the genioglossus muscle oriented superiorly.
FIG. 3 is a schematic anterior depiction of an exemplary general arborization pattern and identification of the bilateral terminal branches of a hypoglossal nerve that innervate the genioglossus muscle (GG), the geniohyoid muscle (GH), and the hyoglossus muscle (HG) of a human. It should be noted that the hypoglossal nerve is illustrated as extending from the anterior mandible to the hyoid bone (i.e. from the top of the page to the bottom).
FIG. 4 is a top view of a neuromodulation device in a non-deployed configuration according to an aspect of the present disclosure.
FIG. 5 is a top view of a neuromodulation device in a fully deployed configuration according to an aspect of the present disclosure. The neuromodulation lead can be inserted either from the left side or the right side of the patient.
FIG. 6 is a schematic anterior view of a neuromodulation lead depicting the location of the electrodes on the lead body of the neuromodulation lead being adjacent to the location of distal branches of the hypoglossal nerve when the neuromodulation lead is fully deployed according to an aspect of the present disclosure. It should be noted that the hypoglossal nerve is illustrated as extending from the anterior mandible to the hyoid bone. (*i*.*e*. from the top of the page to the bottom)
FIG. 7 is a schematic anterior view of a neuromodulation lead depicting the location of the electrodes on the lead body of the neuromodulation lead being adjacent to the location of distal branches of the hypoglossal nerve when the neuromodulation lead is fully deployed according to another aspect of the present disclosure. It should be noted that the hypoglossal nerve is illustrated as extending from the anterior mandible to the hyoid bone. (i.e. from the top of the page to the bottom)
FIG. 8 is a perspective enlarged view of a neuromodulation device according to an aspect of the present disclosure.
FIG. 9 is a flow diagram of exemplary steps of an exemplary method of treating sleep disordered breathing according to an aspect of the present disclosure.
FIGs. 10-15 are schematic illustrations of different views of a neuromodulation device implanted in a patient identifying anterior lingual musculature, the hypoglossal nerve including branches thereof, and surrounding anatomical structures.
FIG. 16 is a transverse view of a neuromodulation device implanted in a patient identifying an illustrative incision and implantation site.

### DETAILED DESCRIPTION

The invention is defined in the claims. As used herein with respect to a described element, the terms "a," "an," and "the" include at least one or more of the described elements including combinations thereof unless otherwise indicated. Further, the terms "or" and "and" refer to "and/or" and combinations thereof unless otherwise indicated. By "substantially" is meant that the shape or configuration of the described element need not have the mathematically exact described shape or configuration of the described element but can have a shape or configuration that is recognizable by one skilled in the art as generally or approximately having the described shape or configuration of the described element. As used herein, "stimulate" or "modulate" in the context of neuromodulation includes stimulating or inhibiting neural activity. A "patient" as described herein includes a mammal, such as a human being. By "improving," the patient's medical disorder is better after therapy than before therapy. As used herein, the terms, "inferior," "superior," "cranial," and caudal refer to anatomical planes and directions when the patient is in a standard anatomical position. Similarly, the terms "left" and "right" refer to the position of elements that correspond to the left and right side of a patient's body in a standard anatomical position.

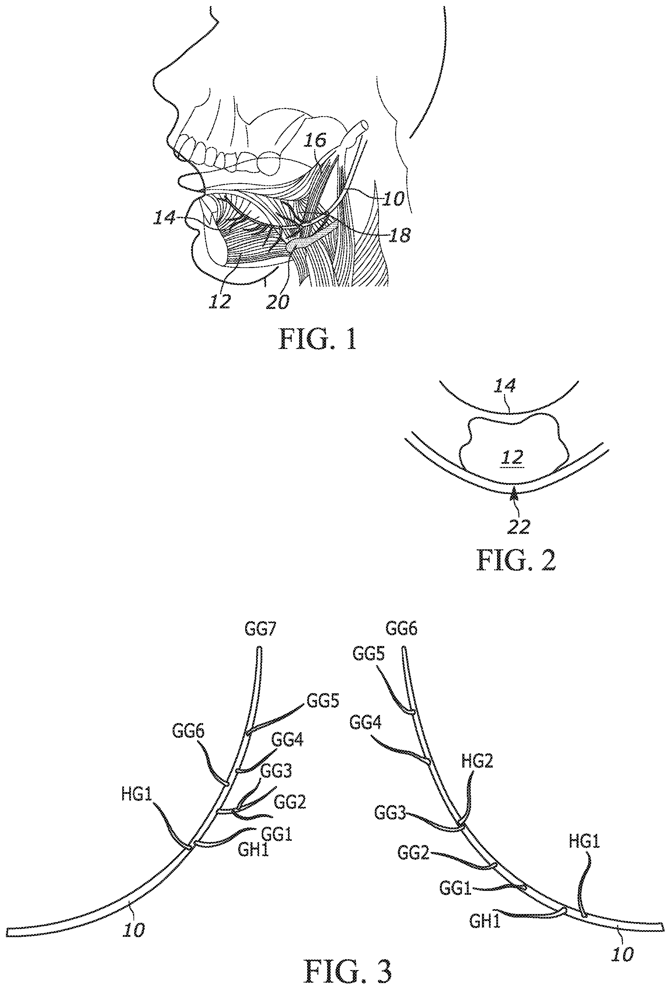

By way of background and with reference to FIG. 1, the hypoglossal nerve **10** controls many upper airway muscles that affect airway patency. The hypoglossal nerve **10** innervates the geniohyoid muscle **12,** the intrinsic muscles of the tongue, and the extrinsic muscles of the tongue, such as the genioglossus muscle **14,** the styloglossus muscle **16,** and the hyoglossus muscle **18.** The genioglossus muscle and the geniohyoid muscles **14** and **12** are primary muscles involved in the dilation of the pharynx. As schematically illustrated in FIG. 2, the geniohyoid muscle **12** is situated superior to the mylohyoid muscle **22** and inferior to the genioglossus muscle **14.** Contraction of the genioglossus muscle **14** can provide tongue protrusion, hence widening of the pharyngeal opening. Activation of the geniohyoid muscle **12** along with a tone present in the sternohyoid muscle can pull the hyoid bone **20** ventrally, thus again dilating the pharynx. On the other hand, the hyoglossus muscle **18** and the styloglossus muscle **16** are considered tongue retractor muscles. As such, the hypoglossal nerve has several branches as it courses toward the anterior lingual muscles, and specifically to the genioglossus muscle. The branches innervate both retruser and protruser muscles of the anterior lingual system. It is believed that effective treatment of SDB requires stimulation of the protruser muscles without activation of the retruser muscles. Thus, for neuromodulation therapy to be effective it is considered important to localize stimulation to the protruser muscles while avoiding activation of the retruser muscles.

Referring back to FIG. 1 and as further schematically illustrated in FIG. 3, as the hypoglossal nerve **10** courses toward the anterior mandible, the first branches of the hypoglossal nerve **10** are branches that innervate the hyoglossus muscle (HG) and styloglossus muscles. The next branches of the hypoglossal nerve **10** are branches that innervate the geniohyoid (GH) and genioglossus (GG) muscles. In general, the number of branches varies individual to individual, but the course and direction of the branches are largely consistent individual to individual.

Although many methods of neuromodulating the anterior lingual muscles have been attempted, it is not known which branches are important to stimulate to achieve the desired efficacy for the treatment of SDB. It is believed that for treatment efficacy, a lead should be configured such that individual or small groups of individual branches can be activated as needed to achieve the desired effect.

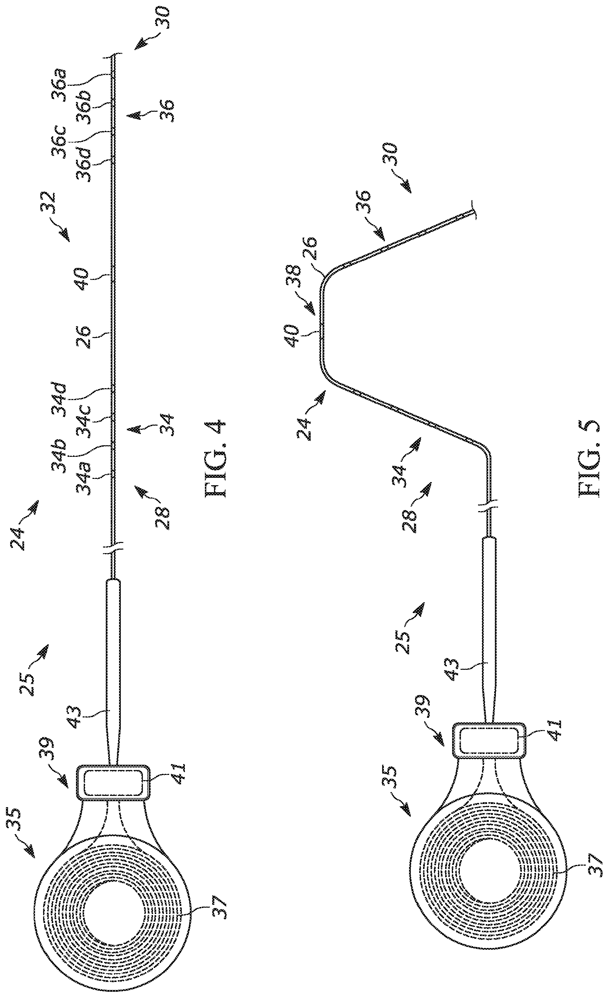

A neuromodulation lead is provided herein that is configured to account for these and other considerations. In particular, a neuromodulation lead is provided that can be inserted and be positioned in the plane between the geniohyoid muscle and the genioglossus muscle. The neuromodulation lead can be configured to position electrodes along the nerve distribution of the hypoglossal nerve and its branches bilaterally. With reference to FIG. 4 and 5, the neuromodulation lead **24** comprises a lead body **26** having a left portion **28** comprising a left set of electrodes **34,** a right portion **30** comprising a right set of electrodes **36,** and an intermediate portion **32** therebetween. As described in more detail below, the electrodes can be used as stimulating electrodes to stimulate neural or neuromuscular tissue and/or as sensing electrodes to sense electrical activity, such as intrinsic or evoked electrical signals. Lead body **26** can be biased towards a substantially omega shape when fully deployed as shown in FIG. 5. In other words, lead body can be configured to transition from a substantially linear shape, as shown in FIG. 4, in a non-deployed state, such as during insertion, to a substantially omega shape, as shown in FIG. 5, when fully deployed. The neuromodulation lead is fully deployed when the neuromodulation lead is implanted in the patient's body and the electrodes are positioned at the desired locations in the patient's body. The omega shape of the neuromodulation lead can be created at the time of manufacturing such that the final form of the lead body is biased to have the omega shape, such bias being overcome if needed during insertion of the lead. The bias can be created, for example, by heat shaping or material shaping or other methods of manufacturing a biased lead. As stated above, the omega bias can allow the lead and the electrodes to mirror the anatomy of the hypoglossal nerve bilaterally as the nerve approaches and starts to branch into the genioglossus muscle.

A left set of electrodes **34** can be disposed on left portion **28** of lead body **26** and a right set of electrodes **36** can be disposed on right portion **30** of lead body **26.** The electrodes can be used as stimulating electrodes. The electrodes can also be used as both stimulating and sensing electrodes for both stimulating target sites as well as sensing electrical activity, such as electromyogram activity, from the anterior lingual muscles. The electrodes can be ring electrodes extending substantially 360°about the lead body, for example, and can have substantially the same size as the target stimulation site(s). The electrodes can also be directional electrodes and not extend 360° about the lead body. Further, the electrodes can have electrode coatings to reduce the signal to noise ratio and/or allow for better long-term recording characteristics if used as sensing electrodes. If used as stimulating electrodes, an electrode coating can also allow the electrodes to have a larger charge injection profile for stimulation safety.

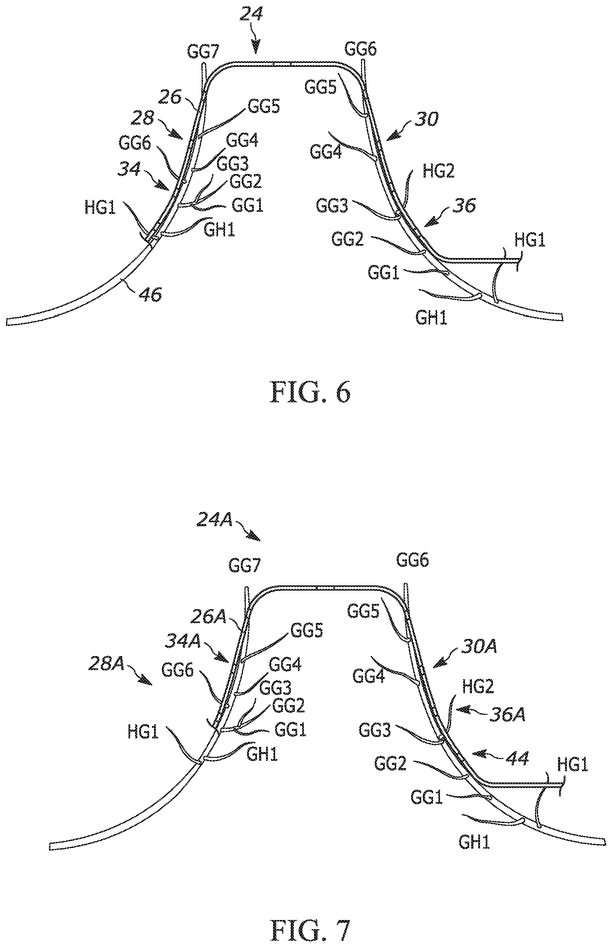

Intermediate portion **32** of lead body **26** can define an apex **38** and an ultrasound marker **40** can be disposed at apex **38.** As such, the neuromodulation lead can be inserted via ultrasound and ultrasound marker **40** can allow the user to identify when the apex of the neuromodulation lead is positioned at midline, allowing the electrode sets **34** and **36** to be positioned along the distribution of the hypoglossal nerve and its branches bilaterally as illustrated in FIG. 6. Ultrasound can also be used to track motion or potential dislodgment of the lead over time. One or more anchors can be disposed on the lead body to secure the neuromodulation lead in place. Such anchors can be hard or soft anchors, for example, including tines, barbs, prefabricated sutures, deployable anchors including time dependent deployable anchors (e.g. anchors that are polymer coated and deploy or release once the polymer dissolves), or combinations thereof.

The lead body can have different shapes. For example, the lead body can be cylindrical, flat or have an oval cross-sectional shape. The lead body can also have enlarged segments to allow for disposition of larger electrode pads or contacts thereon along the length of the lead.

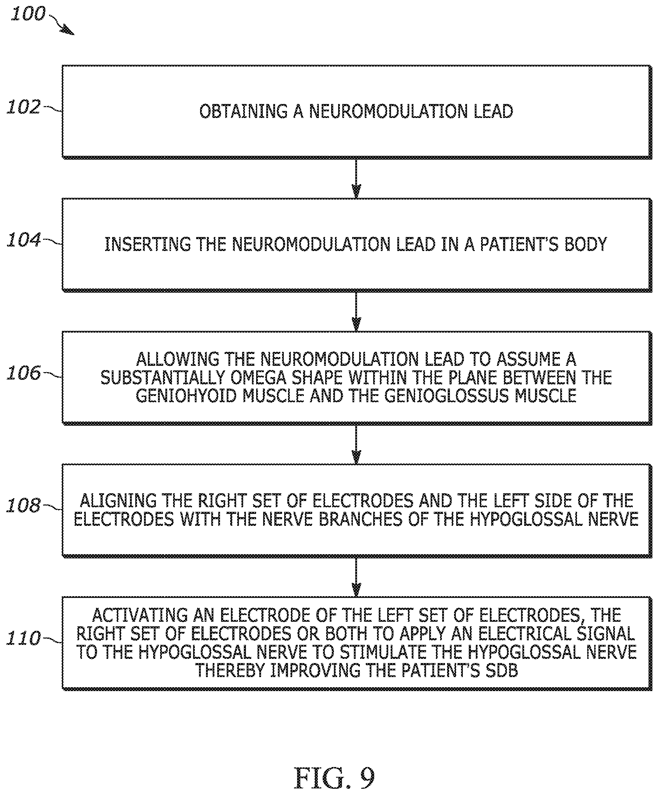

FIGs. 4 and 5 also illustrate an exemplary neuromodulation device **25** comprising neuromodulation lead **24** and including an exemplary power receiver **35** that can be operably coupled to neuromodulation lead **24.** Power receiver **35** can include a coiled receiver antenna **37** configured to produce an induced current in response to being disposed in an electromagnetic field. The antenna comprises a substrate having an upper surface and a lower surface, an upper coil comprising a plurality of coil turns disposed on the upper surface of the substrate, and a lower coil comprising a plurality of coil turns disposed on the lower surface of the substrate. The upper and lower coils are electrically connected to each other in parallel. In particular, advantageously, antenna 37 can implement a unique two-layer, pancake style coil configuration in which the upper and lower coils are configured in parallel. As a result, the upper and lower coils can generate an equal or substantially equal induced voltage potential when subjected to an electromagnetic field. This can help to equalize the voltage of the coils during use, and has been shown to significantly reduce the parasitic capacitance of antenna **37.** In this parallel coil configuration of antenna **37,** upper and lower can be shorted together within each turn. This design has been found to retain the benefit of lower series resistance in a two-layer design while, at the same time, greatly reducing the parasitic capacitance and producing a high maximum power output. Such an antenna is described in more detail in copending U.S. Patent Application No. 16/865,541, entitled: "Implantable Stimulation Systems, Devices and Methods" filed on May 4, 2020 specifically, paragraphs [0043], [0044], and FIGs. 9A and 9B. Neuromodulation device **25** can further comprise electronics package **39** with one or more electronic components **41** mounted therein configured to control the application of stimulation energy via one or more of the electrodes. The antenna can be configured to supply electrical current to the electronics package to power the electronics package. The antenna can be constructed from a flexible circuit board and the upper and lower coils can be etched from conductive layers laminated onto the substrate of the antenna. The electronics package can comprise one or more electronic components mounted on a portion of the flexible circuit board, wherein the flexible circuit board is configured to electrically connect the one or more electronic components to the antenna. The neuromodulation device can include pigtail connector **43,** which can extend from electronics package **39** and can facilitate connecting lead **24** to the electronics package. Pigtail connector **43** can facilitate a detachable connection between electronics package **39** and lead **24** so that leads of different configurations can be connected to the electronics package. This can facilitate manufacturing of the neuromodulation device. This can also allow a physician to select a lead having a desired size and/or configuration.

Additionally, the neuromodulation lead being separate from, and connectable to, the remainder of the neuromodulation device via a pigtail connector, can facilitate implanting the lead separately. As a result, implanting the lead can be much less invasive, allowing the lead to be placed via a small incision. An integrated design may necessitate a larger incision and also the need to handle and manipulate the entire neuromodulation device as a whole during the implantation process, which could complicate the lead placement, as the surgeon could have to work around the remainder of the neuromodulation device, *e*.*g*., the electronics package and the antenna.

As depicted in FIG. 6, the location of left and right set of electrodes **34** and **36** on the respective left and right portion **28** and **30** of lead body **26** can be based on the respective left and right location of a stimulation target site comprising the hypoglossal nerve trunk **46,** distal branches of the hypoglossal nerve such as terminal branches that innervate the genioglossus muscle indicated by reference characters GG1 to GG7 or both when neuromodulation lead is fully deployed. It should be noted that the number of branches depicted in FIG. 6 and other similar figures is only exemplary as the number of branches can vary from individual to individual as stated above. Rather, FIGs. 6-8 are provided to depict the general location of the branches of the hypoglossal nerve. The location of the left and right set of electrodes on the respective left and right portion of the lead body can also be based on the respective left and right location of stimulation sites comprising nerve branches that innervate horizontal fibers within the genioglossus muscle, oblique fibers within the genioglossus muscle, or both when the neuromodulation lead is fully deployed.

As depicted in FIGs. 5 and 6, left set of electrodes **34** and right set of electrodes **36** can be symmetrically disposed on lead body **26.** Alternatively, as depicted in FIG. 7, a neurostimulation lead **24A** can comprise a lead body **26A** where the left set of electrodes **34A** and the right set of electrodes **36A** are asymmetrically disposed on the lead body to avoid the hyoglossus muscle branches, for example. For instance, in an aspect, the electrode configuration of the lead can be such that an electrode is not aligned with a hyoglossus muscle branch when the lead is implanted whereas an electrode is disposed on a distal more section **44** of right portion **30A** of lead body **26A.** Such an embodiment can avoid stimulating the hyoglossus muscle branch HG1, for example, since the hyoglossus muscle is a retruser muscle and given that the location of the hyoglossus muscle branch can be different on the left side of the hypoglossal nerve of a patient compared to the right side of the hypoglossal nerve as illustrated in FIG. 7.

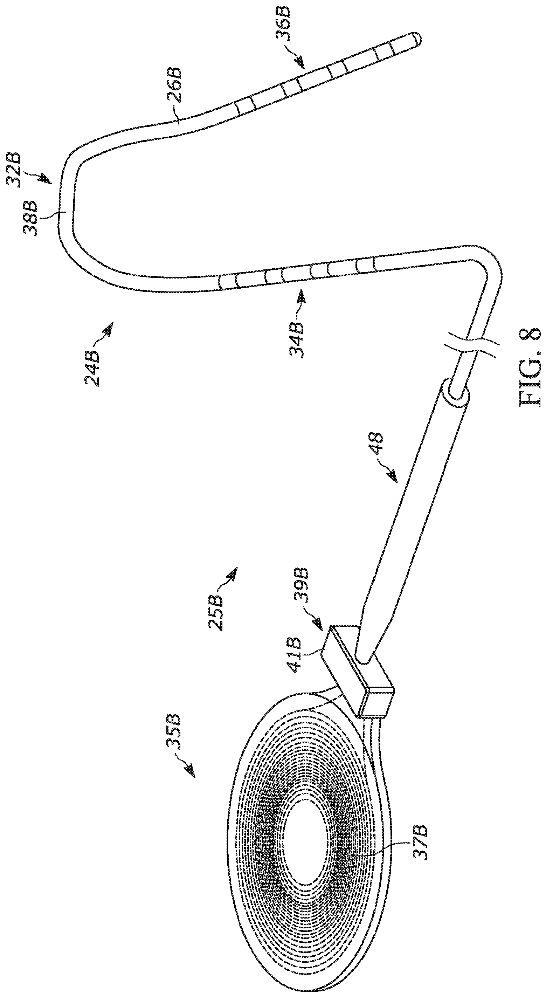

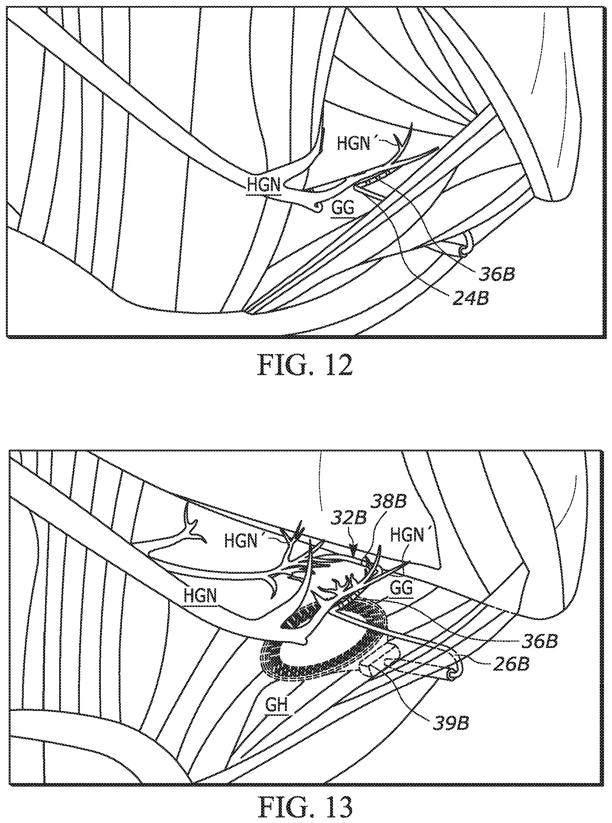

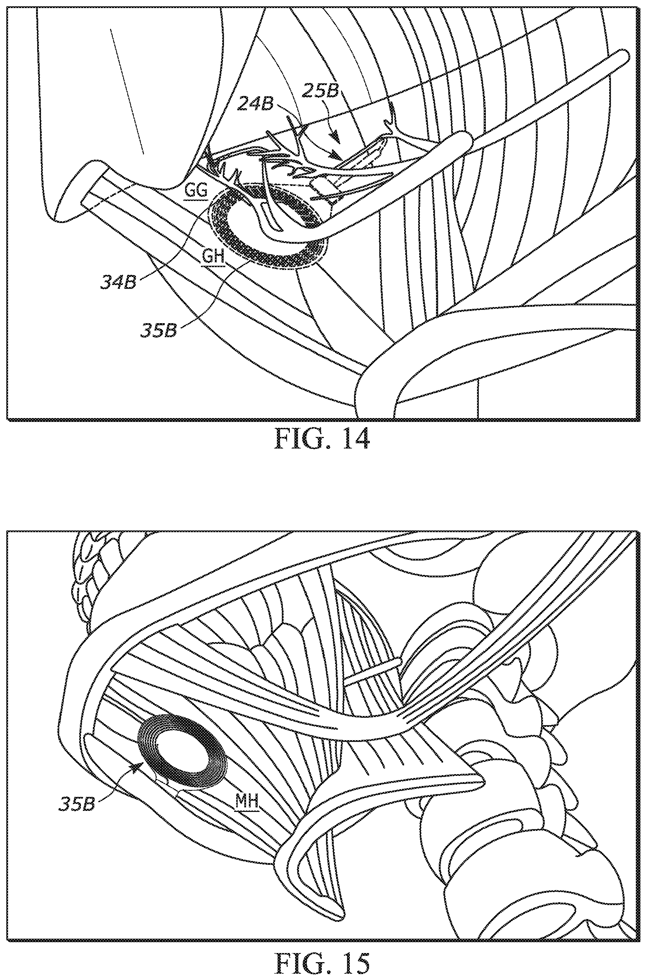

In certain aspects and with respect to FIG. 8 and FIGs. 10-15, when neuromodulation lead **24B** is fully deployed, intermediate portion **32B** of lead body **26B** defining apex **38B** can be located inferior/caudal to the left and right electrode sets **34B** and **36B** respectively. This can be seen in the inferior bend or bias of lead body **26B** that leads into intermediate portion **32B.** Such a bend or bias can allow the lead to exert upward or cranial pressure to press the electrodes against the genioglossal muscle and/or the hypoglossal nerve to allow for better contact between the electrode sets and the hypoglossal nerve and/or genioglossus muscle. In particular, this pressure is created by the intermediate portion, including the apex, being more caudal in the body and allowing the left and right portion of the lead body to be pushed more cranially into the genioglossus muscle. This bias allows for better contact with the genioglossus muscle, more lead stability and hence better long-term performance of the lead.

In particular, such a bias can reduce motion of the lead after encapsulation/scar tissue grows around the lead and thus allow for better contact between stimulating electrodes and the target stimulation sites as well as better contact between sensing electrodes and the muscle(s) from which electrical activity is sensed. The inferior bias can also reduce the amount of encapsulating tissue around the lead as well. This can improve the recording of electromyography (EMG) signals from muscles innervated by the hypoglossal nerve since the more encapsulating tissue around the sensing electrodes, the harder it can be to detect an EMG signal long term. As such, reduced motion of the lead and less encapsulation of tissue around the lead can result in better EMG recording as well as stimulation of target sites at lower stimulation thresholds and more consistent responses from stimulation over time.

Neuromodulation lead **24B** can also be part of a neuromodulation device **25B** that includes a power receiver **35B** and electronics package **39B.** Power receiver **35B** can include a coiled receiver antenna **37B** (exemplary aspects of which are described above).

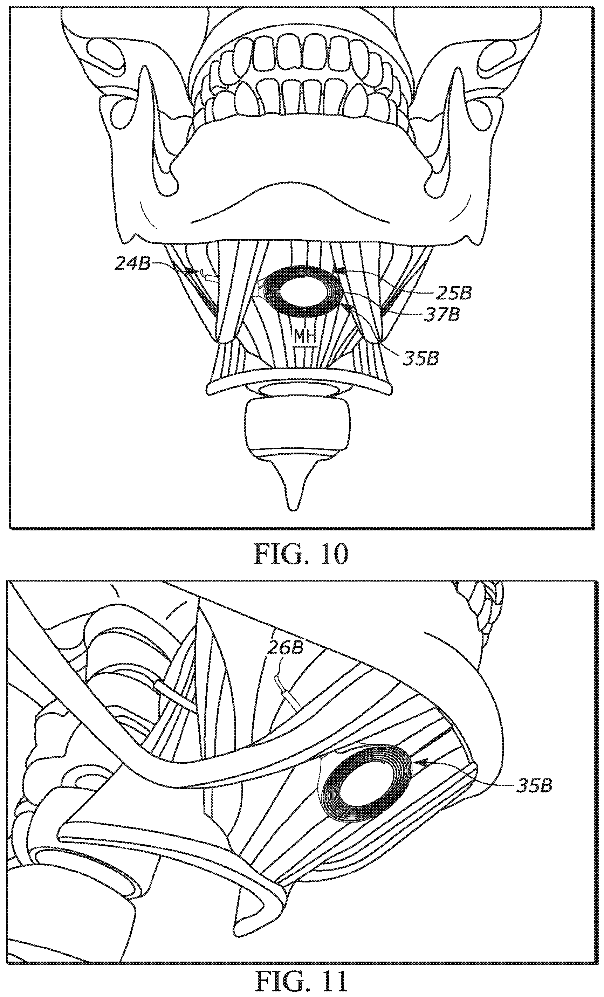

Referring to FIGs. 9 and with reference to FIGs. 10-15, an exemplary method of improving SDB or another disorder is also provided using a neuromodulation lead. Such a method **100** can comprise obtaining a neuromodulation lead **24B (102)** comprising left and right electrodes **34B** and **36B** respectively. FIGs. 10-15 also illustrates an exemplary neuromodulation device **25B** with a power receiver **35B** including a coiled receiver antenna **37B** that is operably coupled to neuromodulation lead **24B.** Method **100** can include inserting the neuromodulation lead in a patient's body **(104)** and allowing the neuromodulation lead to assume a substantially omega shape within the plane between the geniohyoid muscle **GH** and the genioglossus muscle **GG (106).** The hypoglossal nerve **(HGN)** and its branches **(HGN')** are located in this plane bilaterally. Method **100** can further include aligning the right set of electrodes **36B** and the left set of electrodes **34B** with the nerve branches **HGN'** of the hypoglossal nerve **HGN (108).** Method **100** can then comprise activating an electrode of the right set of electrodes **36B,** the left set of electrodes **36B** or both to apply an electrical signal to the hypoglossal nerve **HGN** to stimulate the hypoglossal nerve **HGN** thereby improving the patient's SDB **(110).** The method can also include avoiding stimulating hyoglossus muscle branches, the styloglossus muscle branches, or both. Although the method outlined above is described with respect to the neuromodulation device depicted in FIG. 8, it is understood that other embodiments of a neuromodulation device and neuromodulation lead can be used in such methodology.

Regarding an exemplary method of implanting a neuromodulation device, such a method can comprise inserting a stylet about the neuromodulation lead or into a lumen of the neuromodulation lead such that the neuromodulation lead assumes a substantially linear shape.

The method can further include percutaneously inserting the neuromodulation lead in a patient's body under ultrasound guidance, for example. The method can then comprise positioning the neuromodulation lead, for example, between the geniohyoid muscle and the genioglossus muscle plane of the patient. The method can further include retracting the stylet to allow the neuromodulation lead to assume a substantially omega shape, for example, within the plane between the geniohyoid muscle and the genioglossus muscle. The method can then include activating an electrode of the left set of electrodes, the right set of electrodes or both to apply an electrical signal to a target site to stimulate the hypoglossal nerve, for example, thereby treating a medical disorder such as the patient's SDB. The target site can be, for example, a hypoglossal nerve trunk, distal branches of the hypoglossal nerve, horizontal fibers within an anterior lingual muscle, oblique fibers within an anterior lingual muscle, or combinations thereof. The method can also include avoiding stimulating hyoglossus muscle branches, the styloglossus muscle branches, or both.

With respect to a specific exemplary percutaneous method of implantation, an insertion tool can be used to create a percutaneous puncture through the skin and subcutaneous tissue below the skin. When at the right depth, the insertion tool can create a curved path such that insertion of the neuromodulation lead does not penetrate into the genioglossus muscle but remains within the plane between the geniohyoid muscle and the genioglossus muscle. When positioned correctly, a small instrument can be deployed into the muscle plane and then swept anteriorly and posteriorly in line with the muscle plane to create a small space for the neuromodulation lead to be deployed between the geniohyoid muscle and the genioglossus muscle. Such steps can involve soft, blunt dissection of the muscle plane and not dissection of the muscle fibers. For example, a balloon dissection tool having a pre-configured balloon geometry can be inflated and cause separation between the muscles using gentle pressure. Once space is made for the neuromodulation lead, the small instrument can be retracted and the neuromodulation lead can be deployed. The lead can be deployed across midline, allowing the operator to visualize a midline ultrasound marker. Once positioned correctly, the neuromodulation lead can be deployed by removing the stiffener around or inside the lead, allowing the neuromodulation lead to take its natural omega shape, all within the plane between the geniohyoid and genioglossus. The insertion tool can be removed and the proximal end of the neuromodulation lead can be exposed through the skin.

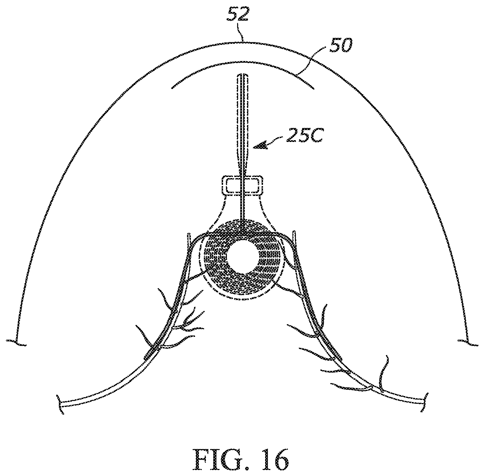

With reference to FIGs. 10 and 16, another exemplary non-percutaneous method of implanting a neuromodulation device **25C** uses a "pull along" lead approach instead of a stylet. Instead of using ultrasound and a percutaneous approach, such a surgical approach can utilize a small incision and dissection to allow visualization of the surgical plane in which the neuromodulation lead **24C** is inserted. The procedure can start with approximately a 1.5 centimeter (cm) to 2 cm transverse incision **50** in the chin **52** fold (*e*.*g*. a sub-malar incision). This incision can be directed through the skin and subcutaneous fat and then, dissecting through the platysma muscle and sub-platysmal fat, the digastric muscles (left and right) can be located and the muscle raphe of the mylohyoid muscle **(MH)** can be identified. Using standard techniques, the mylohyoid raphe can be dissected and the surface of the geniohyoid muscle and its midline raphe can be identified. Again, using standard techniques, the raphe of the geniohyoid can be dissected through to expose a small portion (e.g. approximately 1 cm) of the plane between the geniohyoid muscle and the genioglossus muscle. This plane can be identified by the midline and lateral fat pads that surrounds the nerves and vascular that innervate the anterior lingual muscles (*e.g.* geniohyoid, genioglossus and others). These fat pads are where the hypoglossal nerve trunk and branches are localized.

Then a small curved needle can be inserted just medial to the jaw line and just anterior to the mandibular notch where the facial artery runs. The needle can be inserted with a medial and posterior approach, so that the tip of the needle enters into the plane exposed with the surgical dissection, *e*.*g*. the plane between the geniohyoid and genioglossus muscles. The needle can be inserted into the plane at the caudal border of the exposure and cross midline exiting the plane in a lateral direction. The needle can then exit the skin contralaterally to the original insertion location. The needle can be attached to the neuromodulation lead using a connector, such as a suture. The needle can be pulled through to allow the neuromodulation lead to enter the surgical plane. The connector can be removed and the position of the neuromodulation lead can be adjusted so that the electrodes align with the fat pads and the nerve branches of the hypoglossal nerve. The proximal end of neuromodulation lead can be exposed at the original insertion point and that proximal end can be connected to the electronic package and power receiver once the power receiver antenna coil is implanted. In particular, the power receiver antenna can be implanted in a caudal direction from the original transverse chin fold incision. The power receiver antenna can be implanted deep to the digastric muscles on top of the mylohyoid muscle **(MH).** Once the power receiver antenna is placed, the proximal end of the neuromodulation lead (via a connector, for example) can be tunneled to the power receiver antenna and connected. The muscle planes and the skin can be closed to complete the insertion.

The present invention is defined by the appended claims.

## Claims

1. A neuromodulation lead (24) configured to be placed between a geniohyoid muscle and a genioglossus muscle, the neuromodulation lead comprising:
a lead body (26) having a left portion (28), a right portion (3), and an intermediate
portion (32)
therebetween;
a left set of electrodes (34) disposed on the left portion of the lead body; and
a right set of electrodes (36) disposed on the right portion of the lead body,
wherein the left portion, the right portion, the intermediate portion and the locations of the left and right sets of electrodes respectively on the left and right portions of the lead body are configured to align the left set of electrodes and the right set of electrodes with the left nerve branches and the right nerve branches of a hypoglossal nerve, respectively, when the neuromodulation lead is fully deployed, and wherein the intermediate portion of the lead body is biased towards an inferior position relative to the left and right sets of electrodes when the neuromodulation lead is fully deployed, such that the intermediate portion is configured to push the left and the right sets of electrodes further cranially into the genioglossus muscle.

2. The neuromodulation lead of claim 1, wherein the locations of the left and right sets of electrodes on the respective left and right portions of the lead body are based on the locations of respective left and right stimulation target sites comprising the hypoglossal nerve trunk, distal branches of the hypoglossal nerve, or both when the neuromodulation lead is fully deployed.

3. The neuromodulation lead of any one of claims 1 to 2, wherein the locations of the left and right sets of electrodes on the respective left and right portions of the lead body are based on the locations of respective left and right stimulation sites comprising nerve branches that innervate horizontal fibers within the genioglossus muscle, nerve branches that innervate oblique fibers within the genioglossus muscle, or both when the neuromodulation lead is fully deployed.

4. The neuromodulation lead of any one of claims 1 to 3, wherein the intermediate portion defines an apex of the lead body, the neuromodulation lead further comprising an ultrasound marker disposed at the apex of the lead body.

5. The neuromodulation lead of any one of claims 1 to 4, wherein the left set of electrodes and the right set of electrodes are asymmetrically disposed on the lead body.

6. The neuromodulation lead of claim 5, wherein in a fully deployed configuration, the right set of electrodes comprises a distal most electrode that is more distal than the distal most electrode of the left set of electrodes.

7. A neuromodulation device comprising:
the neuromodulation lead of any one of claims 1 to 6, and
an antenna (37) operably coupled to the neuromodulation lead, the antenna configured to produce an induced current in response to being disposed in an electromagnetic field, the antenna comprising a substrate having an upper surface and a lower surface, an upper coil comprising a plurality of coil turns disposed on the upper surface of the substrate, and a lower coil comprising a plurality of coil turns disposed on the lower surface of the substrate, wherein the upper and lower coils are electrically connected to each other in parallel.

8. The neuromodulation device of claim 7, further comprising an electronics package (39) configured to control application of stimulation energy via one or more of the electrodes, wherein the antenna is configured to supply electrical current to the electronics package to power the electronics package.

9. The neuromodulation device of claim 8, wherein the antenna is constructed from a flexible circuit board, the upper and lower coils being etched from conductive layers laminated onto the substrate, wherein the electronics package comprises one or more electronic components mounted on a portion of the flexible circuit board, wherein the flexible circuit board is configured to electrically connect the one or more electronic components to the antenna.

10. The neuromodulation lead of any one of claims 3 to 6, wherein the left set of electrodes and the right set of electrodes have substantially the same size as the respective left and right stimulation target sites.

## Patentansprüche

1. Neuromodulationsleitung (24), die konfiguriert ist, um zwischen einem Musculus geniohyoideus und einem Musculus genioglossus platziert zu werden, die Neuromodulationsleitung umfassend:
einen Leitungskörper (26) mit einem linken Abschnitt (28), einem rechten Abschnitt (3) und einem Zwischenabschnitt (32)
dazwischen;
einen linken Satz von Elektroden (34), der an dem linken Abschnitt des Leitungskörpers angeordnet ist; und
einen rechten Satz von Elektroden (36), der auf dem rechten Abschnitt des Leitungskörpers angeordnet ist,
wobei der linke Abschnitt, der rechte Abschnitt, der Zwischenabschnitt und die Positionen der linken und rechten Elektrodensätze jeweils auf den linken und rechten Abschnitten des Leitungskörpers konfiguriert sind, um den linken Elektrodensatz und den rechten Elektrodensatz jeweils an den linken Nervenästen und den rechten Nervenästen eines Nervus hypoglossus auszurichten, wenn die Neuromodulationsleitung vollständig angewendet ist, und wobei der Zwischenabschnitt des Leitungskörpers in Richtung einer inferioren Position relativ zu den linken und rechten Elektrodensätzen vorgespannt ist, wenn die Neuromodulationsleitung vollständig angewendet ist, sodass der Zwischenabschnitt konfiguriert ist, um die linken und rechten Elektrodensätze weiter kranial in den Musculus genioglossus zu schieben.

2. Neuromodulationsleitung nach Anspruch 1, wobei die Positionen der linken und rechten Sätze von Elektroden auf den jeweiligen linken und rechten Abschnitten des Leitungskörpers auf den Positionen der jeweiligen linken und rechten Stimulationszielstellen basieren, die den Hypoglossusnervstamm, distale Äste des Nervus hypoglossus oder beides umfassen, wenn die Neuromodulationsleitung vollständig angewendet ist.

3. Neuromodulationsleitung nach einem der Ansprüche 1 bis 2, wobei die Positionen der linken und rechten Elektrodensätze auf den jeweiligen linken und rechten Abschnitten des Leitungskörpers auf den Positionen der jeweiligen linken und rechten Stimulationsstellen basieren, die Nervenäste, die horizontale Fasern innerhalb des Musculus genioglossus innervieren, Nervenäste, die schräge Fasern innerhalb des Musculus genioglossus innervieren, oder beides umfassen, wenn die Neuromodulationsleitung vollständig angewendet ist.

4. Neuromodulationsleitung nach einem der Ansprüche 1 bis 3, wobei der Zwischenabschnitt einen Scheitelpunkt des Leitungskörpers definiert, wobei die Neuromodulationsleitung ferner einen Ultraschallmarker umfasst, der am Scheitelpunkt des Leitungskörpers angeordnet ist.

5. Neuromodulationsleitung nach einem der Ansprüche 1 bis 4, wobei der linke Satz von Elektroden und der rechte Satz von Elektroden asymmetrisch auf dem Leitungskörper angeordnet sind.

6. Neuromodulationsleitung nach Anspruch 5, wobei in einer vollständig angewendeten Konfiguration der rechte Satz von Elektroden eine am weitesten distal gelegene Elektrode umfasst, die weiter distal ist als die am weitesten distal gelegene Elektrode des linken Satzes von Elektroden.

7. Neuromodulationsvorrichtung, umfassend:
die Neuromodulationsleitung nach einem der Ansprüche 1 bis 6, und
eine Antenne (37), die wirksam an die Neuromodulationsleitung gekoppelt ist, wobei die Antenne konfiguriert ist, um als Reaktion auf das Anordnen in einem elektromagnetischen Feld einen induzierten Strom zu erzeugen, die Antenne umfassend ein Substrat mit einer oberen Oberfläche und einer unteren Oberfläche, eine obere Spule, die eine Vielzahl von Spulenwindungen umfasst, die auf der oberen Oberfläche des Substrats angeordnet sind, und eine untere Spule, die eine Vielzahl von Spulenwindungen umfasst, die auf der unteren Oberfläche des Substrats angeordnet sind, wobei die oberen und unteren Spulen elektrisch parallel zueinander geschaltet sind.

8. Neuromodulationsvorrichtung nach Anspruch 7, ferner umfassend eine Elektronikeinheit (39), die konfiguriert ist, um das Anlegen von Stimulationsenergie über eine oder mehrere der Elektroden zu steuern, wobei die Antenne konfiguriert ist, um der Elektronikeinheit elektrischen Strom zuzuführen, um die Elektronikeinheit mit Strom zu versorgen.

9. Neuromodulationsvorrichtung nach Anspruch 8, wobei die Antenne aus einer flexiblen Leiterplatte aufgebaut ist, wobei die obere und die untere Spule aus leitfähigen Schichten geätzt sind, die auf das Substrat laminiert sind, wobei die Elektronikeinheit eine oder mehrere elektronische Komponenten umfasst, die auf einem Abschnitt der flexiblen Leiterplatte montiert sind, wobei die flexible Leiterplatte konfiguriert ist, um die eine oder die mehreren elektronischen Komponenten elektrisch mit der Antenne zu verbinden.

10. Neuromodulationsleitung nach einem der Ansprüche 3 bis 6, wobei der linke Satz von Elektroden und der rechte Satz von Elektroden im Wesentlichen die gleiche Größe wie die jeweiligen linken und rechten Stimulationszielstellen aufweisen.

## Revendications

1. Sonde de neuromodulation (24) conçue pour être placée entre un muscle génio-hyoïdien et un muscle génio-glosse, la sonde de neuromodulation comprenant :
un corps de sonde (26) présentant une partie gauche (28), une partie droite (3) et une partie intermédiaire (32)
entre celles-ci ;
un ensemble gauche d'électrodes (34) disposé sur la partie gauche du corps de sonde ; et
un ensemble droit d'électrodes (36) disposé sur la partie droite du corps de sonde,
dans laquelle la partie gauche, la partie droite, la partie intermédiaire et les emplacements des ensembles gauche et droit d'électrodes respectivement sur les parties gauche et droite du corps de sonde sont conçus pour aligner l'ensemble gauche d'électrodes et l'ensemble droit d'électrodes avec les branches nerveuses gauches et les branches nerveuses droites d'un nerf hypoglosse, respectivement, lorsque la sonde de neuromodulation est entièrement déployée, et dans laquelle la partie intermédiaire du corps de sonde est sollicitée vers une position inférieure par rapport aux ensembles gauche et droit d'électrodes lorsque la sonde de neuromodulation est entièrement déployée, de telle sorte que la partie intermédiaire est conçue pour pousser les ensembles gauche et droit d'électrodes plus loin en direction crâniale dans le muscle génioglosse.

2. Sonde de neuromodulation selon la revendication 1, dans laquelle les emplacements des ensembles gauche et droit d'électrodes sur les parties gauche et droite respectives du corps de sonde sont basés sur les emplacements de sites cibles de stimulation gauche et droit respectifs comprenant le tronc du nerf hypoglosse, les branches distales du nerf hypoglosse, ou les deux, lorsque la sonde de neuromodulation est entièrement déployée.

3. Sonde de neuromodulation selon l'une quelconque des revendications 1 à 2, dans laquelle les emplacements des ensembles gauche et droit d'électrodes sur les parties gauche et droite respectives du corps de sonde sont basés sur les emplacements des sites de stimulation gauche et droit respectifs comprenant des branches nerveuses qui innervent des fibres horizontales à l'intérieur du muscle génioglosse, des branches nerveuses qui innervent des fibres obliques à l'intérieur du muscle génioglosse, ou les deux lorsque la sonde de neuromodulation est entièrement déployée.

4. Sonde de neuromodulation selon l'une quelconque des revendications 1 à 3, dans laquelle la partie intermédiaire définit un apex du corps de sonde, la sonde de neuromodulation comprenant en outre un marqueur à ultrasons disposé à l'apex du corps de sonde.

5. Sonde de neuromodulation selon l'une quelconque des revendications 1 à 4, dans laquelle l'ensemble gauche d'électrodes et l'ensemble droit d'électrodes sont disposés de manière asymétrique sur le corps de sonde.

6. Sonde de neuromodulation selon la revendication 5, dans laquelle, dans une configuration entièrement déployée, l'ensemble droit d'électrodes comprend une électrode la plus distale qui est plus distale que l'électrode la plus distale de l'ensemble gauche d'électrodes.

7. Dispositif de neuromodulation comprenant :
la sonde de neuromodulation selon l'une quelconque des revendications 1 à 6, et
une antenne (37) couplée de manière fonctionnelle à la sonde de neuromodulation, l'antenne étant configurée pour produire un courant induit en réponse à sa disposition dans un champ électromagnétique, l'antenne comprenant un substrat présentant une surface supérieure et une surface inférieure, une bobine supérieure comprenant une pluralité de spires de bobine disposées sur la surface supérieure du substrat, et une bobine inférieure comprenant une pluralité de spires de bobine disposées sur la surface inférieure du substrat, dans laquelle les bobines supérieure et inférieure sont connectées électriquement l'une à l'autre en parallèle.

8. Dispositif de neuromodulation selon la revendication 7, comprenant en outre un boîtier électronique (39) configuré pour commander l'application d'une énergie de stimulation par l'intermédiaire d'une ou de plusieurs des électrodes, dans lequel l'antenne est configurée pour fournir un courant électrique au boîtier électronique pour alimenter le boîtier électronique.

9. Dispositif de neuromodulation selon la revendication 8, dans lequel l'antenne est construite à partir d'une carte de circuit imprimé souple, les bobines supérieure et inférieure étant gravées à partir de couches conductrices stratifiées sur le substrat, dans lequel le boîtier électronique comprend un ou plusieurs composants électroniques montés sur une partie de la carte de circuit imprimé souple, dans lequel la carte de circuit imprimé souple est configurée pour connecter électriquement le ou les composants électroniques à l'antenne.

10. Sonde de neuromodulation selon l'une quelconque des revendications 3 à 6, dans laquelle l'ensemble gauche d'électrodes et l'ensemble droit d'électrodes présentent sensiblement la même taille que les sites cibles de stimulation gauche et droit respectifs.
